# EUROPEAN PATENT SPECIFICATION

(11) **EP 2 636 426 B1**
(45) Date of publication and mention of the grant of the patent: **28.02.2018**
(21) Application number: 13158045.8
(22) Date of filing: 06.03.2013
(51) Int. Cl.: A61N 1/372, A61B 5/00

(54) **RF-powered communication for implantable device**
HF-getriebene Kommunikation für implantierbare Vorrichtung
Communication alimentée en radio fréquence pour dispositif implantable

(30) Priority: 06.03.2012 US 201213413560
(43) Date of publication of application: 11.09.2013
(73) Proprietor: PACESETTER, INC., Sylmar, CA 91392 (US)
(72) Inventor: Poore, John W., South Pasadena, CA 91030 (US); Borzin, Gene A., Simi Valley, CA 93065 (US)
(74) Representative: Noble, Nicholas

(56) References cited:
- EP-A1- 2 361 654
- US-A1- 2002 045 921
- US-A1- 2003 114 898
- US-A1- 2008 046 038
- US-A1- 2008 077 188
- US-A1- 2009 252 042

## Description

### TECHNICAL FIELD

This application relates generally to implantable devices and more specifically, but not exclusively, to RF-powered communication for implantable devices.

### BACKGROUND

Implantable devices may be employed in various applications. For example, an implantable sensing device may perform functions such as sensing blood pressure, sensing cardiac signals, sensing neurological signals, and so on.

In practice, there may be a need to communicate with an implantable device after it has been implanted in a patient. For example, an external monitoring device located in a person's home, a doctor's office, a clinic, or some other suitable location may be used to retrieve information collected by and/or stored in an implanted device. In the case of an implanted blood pressure sensing device, blood pressure readings collected by the implanted device may occasionally (e.g., periodically) be uploaded to an external monitoring device. Similarly, an external programming device located in any of the above locations may be used by a treating physician to change the operating parameters of an implanted device. Such parameters may include, for example, sensing timing and/or thresholds to be used by the implanted device.

In a typical implementation, an implanted device utilizes radiofrequency (RF) telemetry to communicate with an external device. Consequently, the implanted device includes an RF transceiver that transmits and receives RF signals. In such an implementation, however, it is generally desirable to leave the transceiver in a powered-down or low power state as much as possible since the transceiver consumes a relatively large amount of power. Here, it should be appreciated that the replacement of the battery in an implanted device involves a surgical procedure. Hence, long battery life is an important aspect of such a device.

Some types of implanted devices employ a wakeup scheme whereby an implanted device will periodically turn on its transceiver (e.g., its receiver) to determine whether an external device is attempting to establish a communication session. For example, whenever an external device wishes to establish communication with an implanted device, the external device may transmit signals over one or more designated RF channels. Typically, these signals comprise information such as, for example, an identifier (e.g., key) that identifies the external device and/or the implanted device.

Thus, every time the transceiver of the implanted device is turned on (e.g., at defined intervals), the transceiver conducts an RF scan to determine whether an external device is attempting to establish communication with the implanted device. This may involve, for example, performing an identifier (ID) scan that checks each designated RF channel for any signals that comprise a specified identifier. In the event such a message is detected, the implanted device transmits one or more signals (e.g., in accordance with a handshake protocol) to establish communication with the external device. US2009/252042 and EP-A-2361654 disclose implantable medical devices having wakeup circuitry and communications with reduced power consumption. US2008/046038 discloses a local communication network for implantable medical devices which may transition from an "off" state to an "on" state in response to a wakeup signal from a pinging device.

For some types of implantable devices, low power consumption is of upmost importance. For example, it is desirable for very small implantable devices such as sensing devices and satellite pacing devices to remain implanted for as long as possible (e.g., many years). Due to size limitations, however, such devices have relatively small batteries. While the power required for the primary operations (e.g., sensing) performed by the devices may be kept very low, a relatively large amount of power is still used for RF communication with external devices. Consequently, a need exists for techniques for reducing the amount of battery power consumed by an implantable device during RF communication operations.

### SUMMARY

The invention is defined by the device of independent claim 1 and the method of independent claim 8. Preferred embodiments are defined by the dependent claims.

A summary of several sample examples of the disclosure follows. This summary is provided for the convenience of the reader and does not wholly define the breadth of the disclosure. For convenience, the term some examples may be used herein to refer to a single example or multiple examples of the disclosure.

The disclosure relates in some examples to a communication scheme that facilitates reduced battery power consumption in an implantable device. To conserve battery power, a communication circuit of the implantable device is normally decoupled from a battery of the implantable device. The communication circuit is subsequently coupled to the battery (i.e., the communication circuit is powered-up) upon receipt of RF signals at the implantable device. Here, a circuit that controls whether the communication circuit is to be powered-up obtains its power from the received RF signals (e.g., power is magnetically coupled into the implantable device via an RF magnetic field). Consequently, the implantable device is able to perform RF signal monitoring (e.g., RF "sniffing") without using battery power. Rather, battery power is only used for subsequent communication operations once it has been determined that the implantable device is receiving RF signals.

In some embodiments, a communication circuit is powered-up upon detection of RF signals. For example, upon detection of RF signals received via an antenna, a signal may be generated to control a circuit (e.g., a switching circuit) that selectively couples power from a power source to the communication circuit. Thus, the communication circuit will use battery power only after it has been determined that RF signals have been detected at the implantable device. Upon power-up, the communication circuit may then attempt to establish communication with an external device (e.g., in the event the external device was the source of the RF signals).

In some embodiments, a verification circuit obtains power based on (e.g., derived from) received RF signals. Upon power-up, the verification circuit processes received RF signals to determine whether to power-up a communication circuit. For example, in some embodiments, the verification circuit determines whether the received RF signals comprise a defined identifier (e.g., a communication key) that indicates that the RF signals are from a known type of external device. Based on this determination, the verification circuit provides a control signal to selectively couple power from a power source to the communication circuit. Advantageously, the verification circuit does not consume battery power and the communication circuit will be powered-up (and, hence, commence consuming battery power) only after it has been determined that RF signals have been received from a known type of external device. Upon power-up, the communication circuit may then conduct any needed communication with the external device.

### BRIEF DESCRIPTION OF THE DRAWINGS

These and other examples of the disclosure will be more fully understood when considered with respect to the following detailed description, the appended claims, and the accompanying drawings, wherein:
FIG. 1 is a simplified block diagram of an embodiment of a communication system including RF-powered control circuitry in an implantable device;
FIG. 2 is a simplified block diagram illustrating several components of an embodiment of an implantable device that includes an RF-powered detection circuit;
FIG. 3 is a simplified block diagram illustrating several components of an embodiment of an implantable device that includes an RF-powered verification circuit;
FIG. 4 is a simplified diagram of an embodiment of a communication system including RF-powered control circuitry in an implantable device that provides sensing functionality;
FIG. 5 is a simplified flowchart of an embodiment of operations where power is coupled to a communication circuit upon detection of RF signals;
FIG. 6 is a simplified flowchart of an embodiment of operations where a verification circuit is powered by RF signals;
FIG. 7 is a simplified diagram of an embodiment of a communication system including an implantable cardiac device;
FIG. 8 is a simplified diagram of an embodiment of an implantable cardiac device in electrical communication with one or more leads implanted in a patient's heart for sensing conditions in the patient, delivering therapy to the patient, or providing some combination thereof; and
FIG. 9 is a simplified functional block diagram of an embodiment of an implantable cardiac device, illustrating basic elements that may be configured to sense conditions in the patient, deliver therapy to the patient, or provide some combination thereof.

In accordance with common practice the various features illustrated in the drawings may not be drawn to scale. Accordingly, the dimensions of the various features may be arbitrarily expanded or reduced for clarity. In addition, some of the drawings may be simplified for clarity. Thus, the drawings may not depict all of the components of a given apparatus or method. Finally, like reference numerals may be used to denote like features throughout the specification and figures.

### DETAILED DESCRIPTION

The description that follows sets forth one or more illustrative embodiments. It will be apparent that the teachings herein may be embodied in a wide variety of forms, some of which may appear to be quite different from those of the disclosed embodiments. Consequently, the specific structural and functional details disclosed herein are merely representative and do not limit the scope of the disclosure. For example, based on the teachings herein one skilled in the art should appreciate that the various structural and functional details disclosed herein may be incorporated in an embodiment independently of any other structural or functional details. Thus, an apparatus may be implemented or a method practiced using any number of the structural or functional details set forth in any disclosed embodiment(s). Also, an apparatus may be implemented or a method practiced using other structural or functional details in addition to or other than the structural or functional details set forth in any disclosed embodiment(s).

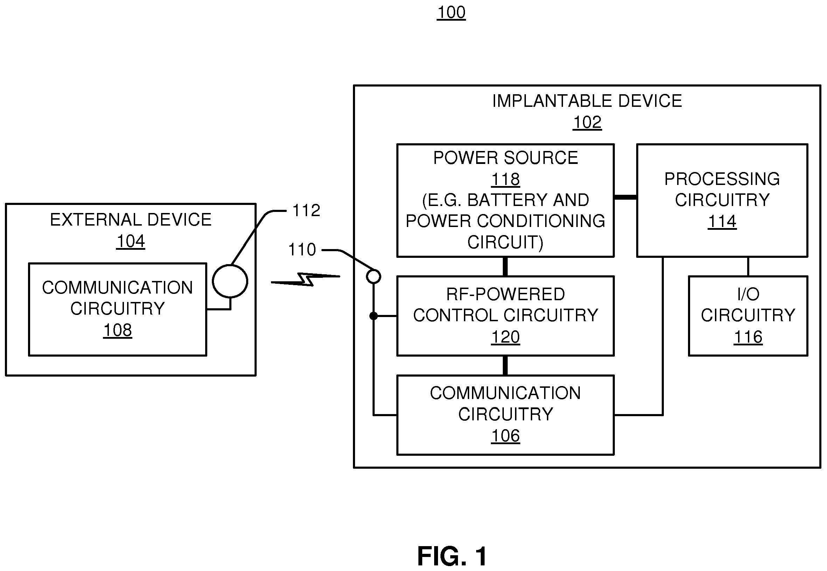

FIG. 1 depicts an embodiment of a communication system 100 where an implantable device 102 communicates via RF signaling with an external device 104. In general, communication circuitry 106 and 108 (e.g., each comprising an RF transceiver, not shown) of the implantable device 102 and the external device 104, respectively, send and receive RF signals via respective antennas 110 and 112.

In a typical implementation, the implantable device 102 is implanted in a patient (not shown) to provide a sensing function, a stimulation function, some other type of implant-related function, or a combination of these functions. The external device 104, in turn, is used to download information to and/or upload information from the implantable device 102. For example, the external device 104 may include programming functionality that programs one or more configuration parameters into the implantable device 102. As another example, the external device 104 may include monitoring functionality that acquires sensed information and/or current parameter information from the implantable device 102.

Processing circuitry 114 and input/output (I/O) circuitry 116 in the implantable device 102 conduct operations that draw power from a power source 118 (e.g., including a battery and a power conditioning circuit). For example, the processing circuitry 114 may operate on information generated by the I/O circuitry 116 (e.g., a sensing circuit) upon sensing a condition within the patient. As another example, the processing circuitry 114 may provide information that is used by the I/O circuitry 116 (e.g., a stimulation circuit) to stimulate patient tissue.

In a typical implementation, the implantable device 102 will not need to communicate with the external device 104 very often. For example, the system 100 may be configured such that the external device 104 initiates communication with the implantable device 102 on a daily, weekly, or some other relatively infrequent basis. Even though such communication does not occur often, this communication may still consume a relatively large amount of power at the implantable device 102 since the communication circuitry 106 may repeatedly process received RF signals to determine whether the external device 104 is attempting to establish communication.

To reduce the amount of power drawn from the power source 118 by communication circuitry 106, the implantable device 102 includes RF-powered control circuitry 120 that keeps the communication circuitry 106 decoupled from the power source 118 most of the time. Here, the control circuitry 120 couples power to the communication circuitry 106 only when it is expected that the external device 104 is attempting to establish communication with the implantable device 102. For example, the control circuitry 120 may be configured to couple the power source 118 to the communication circuitry 106 if RF signals are received at the implantable device 102.

Advantageously, the control circuitry 120 is powered by received RF signals instead of the power source 118. For example, the control circuitry 120 may include rectification circuitry (not shown in FIG. 1) that rectifies RF signals received via the antenna 110. The resulting rectified signal may then be used to power any circuitry that controls the coupling of the power source 118 to the communication circuitry 106.

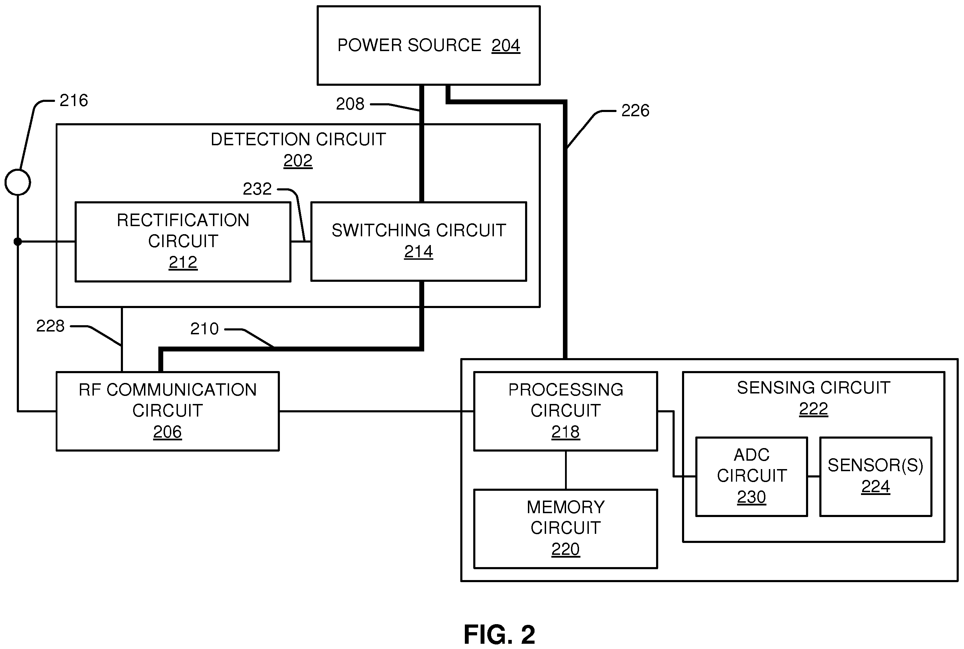

The received RF signals may be used in different ways in different implementations to control this coupling. As described in more detail below in conjunction with FIG. 2, in some embodiments, the received RF signals are used to control a circuit (e.g., a switching circuit) that selectively couples the power source 118 to the communication circuitry 106. For example, upon detection of an RF signal (e.g., upon receipt of RF signals of sufficient magnitude), a switch may be turned on to couple the power source 118 to the communication circuitry 106.

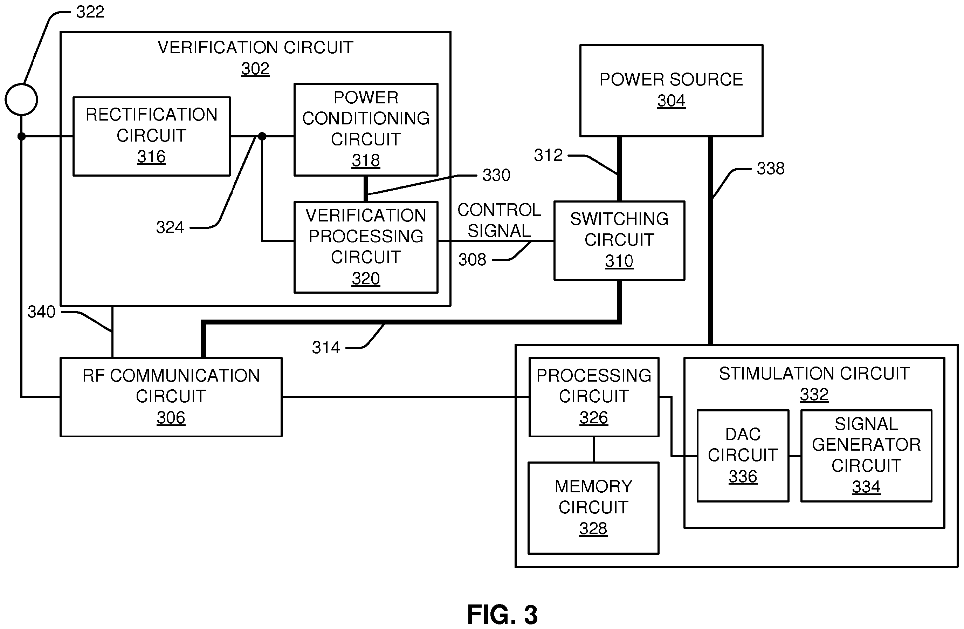

As described in more detail below in conjunction with FIG. 3, in some embodiments, the received RF signals are used to power a verification circuit that processes the received RF signals to determine whether an external device is attempting to establish communication with the implantable device 102. Based on this determination, the verification circuit selectively couples the power source 118 to the communication circuitry 106. For example, the verification circuit may determine whether the received RF signals comprise a defined identifier (e.g., a communication key). If so, the verification circuit couples the power source 118 to the communication circuitry 106.

Through the use of a communication scheme as taught herein, a significant reduction in communication-related battery consumption may be achieved. For example, all RF "sniffing" operations may be performed using power obtained from received RF signals. Only when an RF signal is received (and, optionally, verification performed), is the communication circuitry 106 coupled to the power source 118 and powered-up. Thus, battery power that would otherwise be consumed to conduct periodic RF "sniffing" operations is not consumed in implantable devices that employ a communication scheme as disclosed herein. Moreover, in some embodiments, battery power that would otherwise be wasted processing signals from non-verified sources (e.g., RF interference) is not consumed in implantable devices that employ a communication scheme as disclosed herein.

Such a communication scheme may be advantageously employed in an implantable device that has relatively low power consumption. For example, the processing circuitry 114 and the I/O circuitry 116 may conduct periodic sensing operations or other operations that draw a relatively small amount of power from the power source 118. In such a case, it is possible that the implantable device 102 may remain implanted in a patient for a very long time (e.g., 10 - 15 years or longer) provided that the communication circuitry 106 does not consume too much power from the power source 118. As a specific example, the need for very small implantable medical devices (IMDs) such as sensors and satellite pacers is increasing as technology advances permit small, low power highly integrated electronics including high density, very low power integrated circuits (ICs).

Although the power used to operate such devices is very small, the power associated with transmitting data to or from such a device may be relatively high, limited in some examples by physical laws and ambient electromagnetic noise. As discussed above, one source of power consumption relates to listening or "sniffing" for commands from external devices (e.g., interrogation devices and/or programming devices). Here, the greater the depth of implant, the greater the power required for the RF receiver circuit that "sniffs" (e.g., periodically monitors) for externally generated commands. In practice, implantable sensors or satellite pacing devices may need to be deeply implanted in a patient (in contrast with subcutaneously implanted conventional pacers that employ leads going to the heart). Such a deep implant results in a greater distance between the IMD and the external device and, consequently, more energy being used for communication between these devices.

A specific example of power consumption for sensing operations and conventional telemetry operations follows. This example involves a wireless pressure transducer that is placed inside a patient (e.g., inside the heart). Here, it is assumed that the transducer captures 30 seconds of stored pressure waveforms which will be downloaded once a day (e.g., at 8 Kbits/second) for 15 years. In a sample implementation, telemetry operations and recording the data uses 1 milliamp for approximately 4 seconds per day. Thus, approximately 6 milliamp-hours will be consumed to generate and upload this data over a period of 15 years.

The current consumption for RF "sniffing" operations will be calculated assuming that the RF receiver turns on for a duration of one millisecond every 30 seconds. In a sample implementation, 200 microamperes are consumed during each "sniffing" or listening operation. Accordingly, the total energy cost over 15 years is approximately 13 milliamp-hours.

For a crystal oscillator/counter that runs on 66 nanoamps, the total energy cost over 15 years is approximately 9 milliamp-hours. Thus, the total energy cost for the wireless pressure transducer is approximately 29 milliamp-hours. Assuming a self-discharge rate for the battery of 2%/year, the wireless pressure transducer would require a battery having a capacity on the order of 40 milliamp-hours.

The use of a communication scheme as taught herein reduces the amount of IMD battery power that is consumed for RF communication. An antenna (e.g., a coil) in the IMD receives AC magnetically coupled RF energy from the external device, and uses the received RF energy frequency and/or morphology as a command to, for example, change parameters of the IMD or to invoke data transmission from the IMD. Energy for transmission of a response and/or data to the external device may come, magnetically, from the external device or may come from the IMD battery. In either case, the IMD does not need to draw current from a battery to "sniff" for RF transmissions (e.g., commands) from an external device.

Continuing with the example set forth above, in a case where "sniffing" operations do not consume battery power, the energy requirements for the wireless pressure transducer would be approximately 15 milliamp-hours. Following adjustment for a self-discharge rate of 2%/year, the wireless pressure transducer would require a battery having a capacity on the order of 20 milliamp-hours.

Thus, a very small primary battery may be employed in this case. For example, the dimensions of such a battery may be on the order of 3 millimeters in diameter by 8 millimeters in length.

The use of a communication scheme as taught herein also provides benefits when using rechargeable batteries. For example, these batteries would not need to be recharged as often in this case as compared to a system where RF "sniffing" relied solely on battery power.

FIG. 2 illustrates several components of an embodiment of an implantable device that employs a detection circuit 202. Most of the time (e.g., when the implantable device is not communicating with an external device), the detection circuit 202 is in a state that decouples a power source 204 from an RF communication circuit 206. When the detection circuit 202 detects the reception of RF signals, however, the detection circuit 202 couples power from the power source 204 to the communication circuit 206. In the example of FIG. 2, this involves coupling a power terminal 208 (e.g., power path) of the power source 204 with a power terminal 210 of the communication circuit 206.

The detection of RF signals may be accomplished in different ways in different implementations. In some cases, detection involves generating an output signal in the event the received RF signal is of a certain magnitude (e.g., large enough to cause conduction through a rectifier diode). In some cases, detection is indicated when a received RF signal is of a sufficient magnitude to cause activation of a switch (e.g., a semiconductor switch comprising a transistor such as a FET). In a typical implementation, a received RF signal is coupled to a rectifier, and the output of a rectifier is coupled to a comparator associated with a reference level. Detection of the RF signal is then indicated by a change in the output of the comparator (e.g., where the output controls a FET switch to selectively couple power from the power source 204).

The detection circuit 202 is depicted as including a rectification circuit 212 and a switching circuit 214. It should be appreciated, however, that in some implementations the rectification and switching operations may be performed (fully or in part) by a common circuit.

The rectification circuit 212 (e.g., comprising a rectifier) rectifies RF signals received via an antenna 216 and provides a rectified output signal on a signal path 232. In some implementations, the rectification involves some form of power conditioning (e.g., simple capacitive filtering or more robust filtering) to generate a substantially DC rectified signal.

In general, in the absence of a rectified signal on the signal path 232, the switching circuit 214 is in an "off" state, thereby presenting an "open circuit" (e.g., a high resistance path) between the power terminals 208 and 210. Once a rectified signal is induced on the signal path 232, the switching circuit 214 switches to an "on" state, thereby presenting a "closed circuit" (e.g., a low resistance path) between the power terminals 208 and 210.

The communication circuit 206 is powered-up as a result of being coupled to the power source 204. Upon power-up, the communication circuit 206 attempts to establish communication with an external device (not shown in FIG. 2). For example, in some implementations, the communication circuit 206 may monitor for communication signals (RF signals) received via the antenna 216 and attempt to verify whether a known type of external device (e.g., an authorized programmer device) is sending the RF signals. This verification may involve, for example, determining whether the RF signals comprise a defined identifier (e.g., key) and, in some cases, conducting a communication session commencement handshaking operation.

If a communication session is established with the external device, the communication circuit 206 may cooperate with other components of the implantable device to conduct additional communication-related operations with the external device. For example, the communication circuit may cooperate with a processing circuit 218 (with an associated memory circuit 220 and clocking circuitry, not shown) to upload information to the external device and/or download information (e.g. commands and/or parameters) to the implantable device.

FIG. 2 illustrates an embodiment where the implantable device includes a sensing circuit 222 including at least one sensor 224 (e.g., a blood pressure sensor, a cardiac signal sensor, a neurological signal sensor, etc.) for sensing one or more conditions of a patient. In this example, an analog-to-digital converter (ADC) circuit 230 converts analog signals generated by the sensor 224 into digital information to provide sensed information that is sent to the processing circuit 218 for subsequent processing and/or that is stored in the memory circuit 220. At some point in time, the sensed information (e.g., after processing) may be sent to the communication circuit 206 for transmission to the external device.

The example of FIG. 2 also illustrates that in some embodiments at least one circuit is directly powered (e.g., continually powered) by the power source 204 while at least one other circuit is selectively powered (e.g., power is dynamically enabled and disabled) by the power source 204. Specifically, the processing circuit 218, the memory circuit 220, and the sensing circuit 222 are directly powered by the power source 204 as indicated by a power terminal 226 from the power source 204. Thus, the coupling of the power source 204 to these components does not include the detection circuit 202. Conversely, the communication circuit 206 is selectively powered by the power source 204 under the control of the detection circuit 202. It should be appreciated that in other embodiments other types of circuits may be directly powered or selectively powered as taught herein.

At some point in time, the switching circuit 214 will revert back to its "off" state. As represented by a signal path 228, the communication circuit 206 (or optionally the processing circuit 218 via another signal path, not shown) sends a control signal to the detection circuit 202 to cause the switching circuit 214 to decouple the power source 204 from the communication circuit 206. For example, the switching circuit 214 may have a latching characteristic whereby, once the switching circuit 214 is turned "on," it remains "on" until it is reset. The control signal may be sent, for example, upon determining that the communication with the external device has terminated.

In the embodiment of FIG. 2, in some cases RF signals (e.g., interference) will be detected at the implantable device and result in the communication circuit 206 being powered-up even though the RF signals were not sent by an external device that is authorized to communicate with the implantable device. In such a case, battery power may be wasted. Such RF signals may be generated by, for example, an RFID system, an air conditioner, an implantable device programmer that is not authorized to communicate with the implantable device, or some other RF signal source.

FIG. 3 illustrates several components of an embodiment of an implantable device that employs a verification circuit 302 to control whether a power source 304 is coupled to an RF communication circuit 306. In a typical implementation, the verification circuit 302 determines whether RF signals are being received from an external device that is attempting to communicate with the implantable device (e.g., a programmer that is authorized to communicate with the implantable device). Based on this determination, the verification circuit 302 generates a control signal on a signal path 308 to control a switching circuit 310.

Most of the time (e.g., when the implantable device is not communicating with an external device), this control signal is in a state that causes the switching circuit 310 to decouple the power source 304 from the communication circuit 306. For example, the control signal may set the switching circuit 310 to an "off" state in this case. When the verification circuit 302 verifies the received RF signals, however, the control signal is set to a state that causes the switching circuit 310 to couple power from the power source 304 to the communication circuit 306. For example, the control signal may switch the switching circuit 310 to an "on" state in this case. In the example of FIG. 3, this involves coupling a power terminal 312 of the power source 304 with a power terminal 314 of the communication circuit 306.

Advantageously, the verification circuit 302 is powered by received RF signals. Thus, battery power is not consumed during the verification operation. Accordingly, the embodiment of FIG. 3 may be less susceptible to interference as compared to the embodiment of FIG. 2 in that the embodiment of FIG. 3 may not consume as much battery power when subjected to RF interference.

The verification circuit 302 is depicted as including a rectification circuit 316, a power conditioning circuit 318, and a verification processing circuit 320. It should be appreciated that in some implementations two or more of these operations may be performed (fully or in part) by a common circuit.

The rectification circuit 316 (e.g., comprising a rectifier) rectifies RF signals received via an antenna 322 and provides a rectified output signal on a signal path 324. The power conditioning circuit 318 performs power conditioning (e.g., simple capacitive filtering or more robust filtering) on the rectified signal to provide DC power for the verification processing circuit 320 via a power path 330 (e.g., a conductive trace). In some implementations, the power conditioning circuit 318 includes an energy storage circuit (e.g., a capacitor and/or a rechargeable battery) that stores energy that may be used to provide power for one or more circuits when RF signals are not being received at the implantable device.

Upon obtaining power from the received RF signals, the verification processing circuit 320 is powered-up (e.g., activated) and commences processing RF signals received via the antenna 322 to determine whether the RF signals are from an external device that is attempting to communicate with the implantable device. These received RF signals may comprise the rectified signal provided on the signal path 324, RF signals received from the antenna 322 via another signal path (e.g., a signal path directly from the antenna 322, not shown), or RF signals received in some other manner (e.g., via another antenna, not shown).

Thus, in different implementations or under different operating conditions, the RF signals processed by the verification processing circuit 320 may or may not be the same RF signals (e.g., temporally) that are used to generate power for the verification processing circuit 320. In some cases, the verification processing circuit 320 processes the same RF signals that were used to generate power for the verification processing circuit 320 (e.g., the rectified signal on signal path 324). Thus, in some cases, some of the signal power in an RF signal comprising a key may be used to power the verification circuit that verifies the key. In some cases, the verification processing circuit 320 processes a portion (e.g., a short burst) of the RF signals that were used to generate power for the verification processing circuit 320. In some cases, the verification processing circuit 320 processes different RF signals (e.g., RF signals that arrived later in time) than those that were used to generate power for the verification processing circuit 320. For example, the verification circuit 302 may use a first portion of the received RF signals to obtain power and then process a second portion of the received RF signals to provide the control signal. Here, the circuitry that provides the control signal may use (i.e., may be powered by) stored power that was obtained from the first portion of the received RF signal.

The verification operation performed by the verification processing circuit 302 may take different forms in different implementations. In some implementations, the verification operation involves determining whether the received RF signals comprise a defined identifier. Such an identifier may take the form of, for example, a communication key that is associated with an authorized external device (e.g., a programmer device) and/or the implantable device. Thus, upon receipt of this key, the verification processing circuit 320 is able to make a determination that the RF signals are from an authorized external device and that communication is to be established with that external device.

In some implementations, the received RF signal takes the form of a series of pulses (e.g., generated using on-off keying or some other technique). For example, the identifier (or key) may be indicated by a defined sequence of pulse positions (in time) in the received RF signals. Thus, in some embodiments, the verification process (i.e., the processing of the RF signals) involves determining whether the received RF signals comprise a defined pulse position sequence.

Based on the results of the verification process, the verification processing circuit 320 controls (e.g., adjusts, if applicable) the state (e.g., value) of the control signal provided on the signal path 308 to selectively enable the coupling of power from the power source 304 to the communication circuit 306.

Thus, if the received RF signals pass verification, the communication circuit 306 is powered-up as a result of being coupled to the power source 304. Upon power-up, the communication circuit 306 attempts to establish communication with an external device (not shown in FIG. 3). For example, in some implementations, the communication circuit 306 may monitor for communication signals (RF signals) received via the antenna 322 and further attempt to verify whether a known type of external device (e.g., an authorized programmer device) is sending the RF signals. This verification may involve, for example, conducting a communication session commencement handshaking operation. In some implementations, the communication circuit 306 may simply commence transmissions to the external device (e.g., sensed information may be automatically uploaded to the external device).

If a communication session is established with the external device, the communication circuit 306 may cooperate with other components of the implantable device to conduct additional communication-related operations with the external device. For example, the communication circuit 306 may cooperate with a processing circuit 326 (with an associated memory circuit 328 and clock circuitry) to upload information to the external device and/or download information (e.g. commands and/or parameters) to the implantable device.

FIG. 3 illustrates an embodiment where the implantable device includes a stimulation circuit 332 including a signal generator circuit 334 (e.g., at least one pulse generator, etc.) for stimulating tissue of a patient. In this example, a digital-to-analog converter (DAC) circuit 336 converts stimulation data provided by the processing circuit 326 and/or the memory circuit 328 to an analog signal and provides the analog signal to the signal generator circuit 334. Here, one or more parameters that specified the stimulation data may have been provided by the external device via the communication circuit 306. It should be appreciated that in some implementations the embodiment of FIG. 3 may instead, or in addition, employ sensing circuitry as described herein (e.g., at FIG. 2). Similarly, in some implementations, the embodiment of FIG. 2 may instead, or in addition, employ stimulation circuitry as described herein (e.g., at FIG. 3).

The example of FIG. 3 also illustrates that in some embodiments at least one circuit is directly powered (e.g., continually powered) by the power source 304 while at least one other circuit is selectively powered (e.g., power is dynamically enabled and disabled) by the power source 304. Specifically, the processing circuit 326, the memory circuit 328, and the stimulation circuit 332 are directly powered by the power source 304 as indicated by a power terminal 338 from the power source 304. It should be appreciated that in other embodiments other types of circuits may be directly powered or selectively powered as taught herein.

At some point in time, the switching circuit 310 will revert back to its "off" state. As represented by a signal path 340, the communication circuit 306 (or optionally the processing circuit 326 via another signal path, not shown) sends a control signal to the verification circuit 302 to cause the verification circuit 302 to change the value of the control signal provided on the signal path 308. In this way, the switching circuit 310 will decouple the power source 304 from the communication circuit 306. As above, the control signal provided on the signal path 340 may be sent, for example, upon determining that the communication with the external device has terminated.

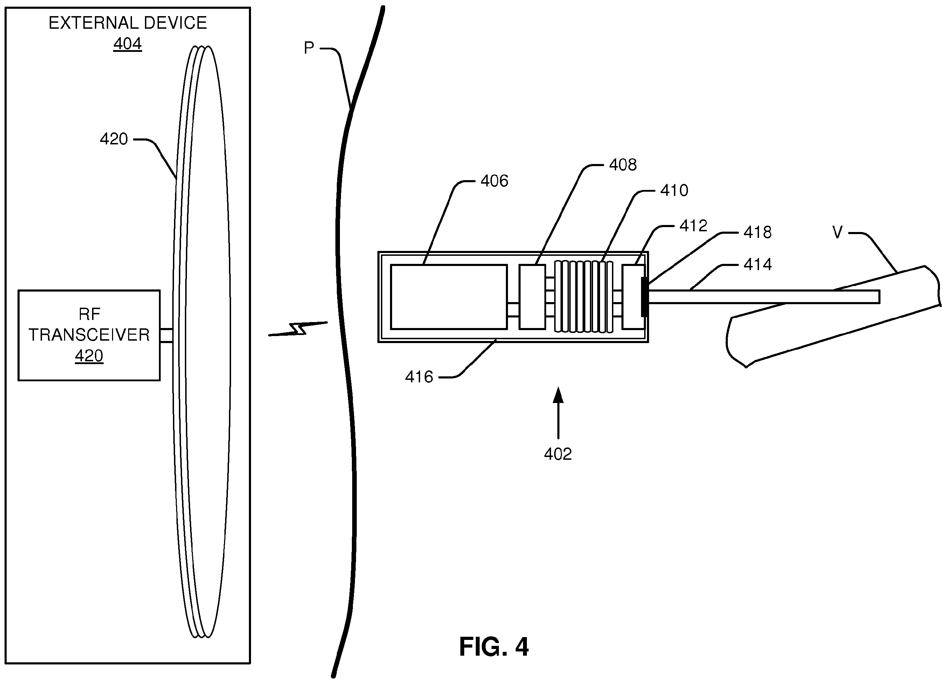

As mentioned above, the teachings herein may be employed to provide an implantable device of a very small size. FIG. 4 illustrates several components of an implantable blood pressure sensor device 402 that is implanted in a patient P and communicates with an external device 404.

The sensor device 402 includes a battery 406, control circuitry 408, an antenna 410, a pressure transducer 412, a thin protruding tube 414, and a biocompatible housing 416. In the example, of FIG. 4, the tube 414 is inserted into a blood vessel V (e.g., a vein, an artery, a chamber, etc.) of the patient P to measure blood pressure at that location. The sensor device 402 may fixed in place within the patient P by means of sutures, an active fixation device, passive fixation, or some suitable fixation technique (fixation means not shown in FIG. 4).

In some implementations, the sensor device 402 has dimensions on the order of: 7 - 8 millimeters in diameter or less and 10 millimeters in length or less. Thus, the sensor device 402 may be implanted into a patient using so-called minimally invasive techniques (e.g., subcutaneous injection techniques or venous techniques). In some implementations, the sensor device 402 has dimensions on the order of: 5 - 6 millimeters in diameter thus facilitating implant into a patient using a so-called venous approach. Typically, the battery of an implantable device takes up most of the space in the device. The above described device may accommodate, for example, dimensions for the battery 406 on the order of: 3 - 5 millimeters in diameter or less and 8 millimeters in length or less. Accordingly, as discussed herein, through the use of a communication scheme taught herein, the sensor device 402 may remain implanted for a very long period of time (e.g., on the order of 10 - 15 years). Moreover, the control circuitry 408 may wake up several times a day (e.g., once every 2 hours) to take measurements, store the sensed information in a memory circuit (in the control circuit 408), and occasionally upload the sensed information to an external device (e.g., on a daily, weekly, monthly, or some other basis). Thus, very detailed blood pressure profiles may be obtained over a long period of time after performing only a single surgical procedure.

The control circuitry 408 provides functionally such as, for example, the communication, control, detection, power conditioning, processing, memory, conversion, verification, clock, and switching functionality described above. In a typical implementation the control circuitry 408 is implemented as an application-specific integrated circuit (ASIC).

In a typical implementation, the antenna 410 comprises a coil. Such an antenna may have dimensions on the order of, for example: 3 - 4 millimeters or less in diameter and 3 - 5 millimeters or less in length.

The pressure transducer 412 includes a flexible diaphragm 418 or some other suitable component that is able to detect pressure that is transferred via the tube 414 (or some other suitable structure). For example, the flexible diaphragm 418 may be in fluid communication with the interior space of the tube 414 (e.g., which may be filled with a gel) such that pressure induced in the vessel V is imparted via the interior space of the tube 414 to the flexible diaphragm 418. The flexible diaphragm 418 may be constructed of various types of biocompatible materials including, for example, silicone, polyurethane, titanium, and so on. For example, the pressure transducer 412 may comprise a thin titanium diaphragm with an attached strain gauge that is coupled to a bridge circuit that generates signals representative of the deflection of the flexible diaphragm 418.

The external device includes an antenna 420 (e.g., a coil) that is much larger than the antenna 410. For example, the antenna 420 may have dimensions on the order of 12 - 20 centimeters in diameter. In this way, an RF transceiver 420 of the external device 404 is able to more effectively exchange information with an RF transceiver (e.g., implemented in the control circuitry 408) of the sensor device 402. Here, RF signals from the transceiver 420 are coupled to the antenna 420 thereby generating a relatively strong RF magnetic field that projects into the body of the patient P. Due in part to the large size of the antenna 420, the RF magnetic field is strong enough to induce a voltage on the relatively small antenna 410, thereby providing RF signals at the control circuitry 408 (e.g., including detection circuitry or verification circuitry as taught herein). Conversely, RF signals from the control circuitry 408 are coupled to the antenna 410 thereby generating a RF magnetic field that projects out of the body of the patient P. Due in part to the large size of the antenna 420, the corresponding voltage induced on the antenna 420 is strong enough to be detected by the transceiver 420.

As discussed herein, in some embodiments, the voltage induced at the antenna 410 is used to trigger detection circuitry (e.g., a semiconductor switch) of the control circuitry 408 and thereby connect the battery 406 to communication circuitry (including the transceiver) of the control circuitry 408. Upon power-up, this transceiver commences communication with the transceiver 420.

In some embodiments, the voltage induced at the antenna 410 is used to power verification circuitry (e.g., a receiver circuit) of the control circuitry 408, whereby the verification circuitry demodulates the received signal and confirms a communication link. Upon confirmation of the communication link, the battery 406 is coupled to the communication circuitry of the control circuitry 408 to enable the communication circuitry to communicate with the external device 404. As mentioned above, this embodiment is used in some examples to prevent interference signals (e.g., stray electromagnetic radiation) from potentially triggering unnecessary power-ups of the communication circuitry of the sensor device 402.

In the embodiments described herein, power and/or information may be coupled via a given antenna. For example, the antenna 410 receives both power and commands from the antenna 420. Conversely, the antenna 420 receives information (e.g., sensed information) from the antenna 410.

Different technologies may be employed to provide the RF signaling between the sensor device 402 and the external device 404 in different embodiments. For example, some embodiments employ a reflective impedance scheme whereby, for example, a transmitted RF signal may be modulated (e.g., by turning the RF signal on and off) by the information (e.g. commands or sensed information) being transmitted. Such a scheme may provide an efficient way (e.g., from a battery power consumption standpoint) for the sensor device 402 to transmit RF signals. In some implementations, a frequency in the range of 100 KHz to 2 MHz is used for the RF transmissions.

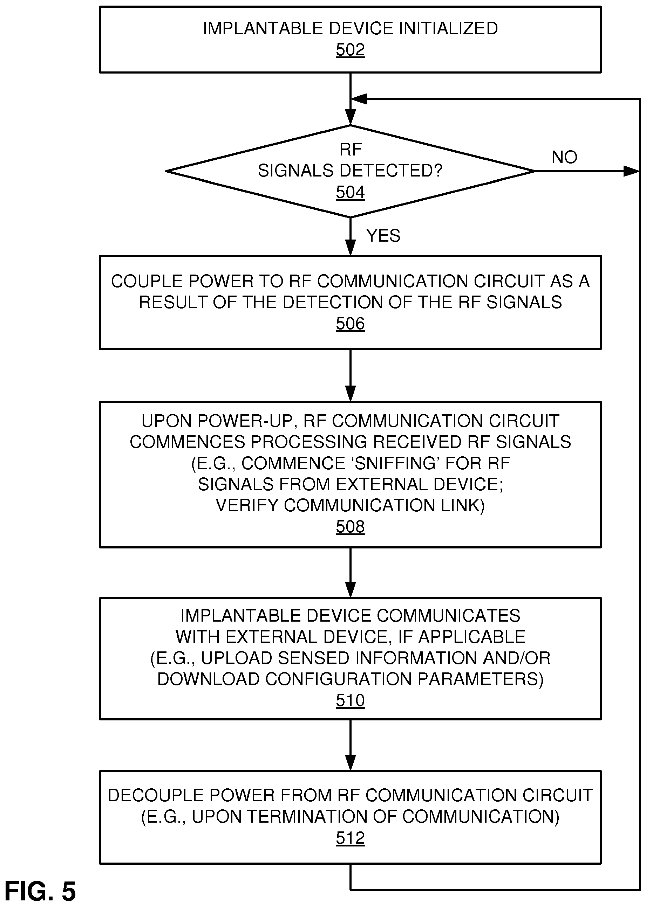

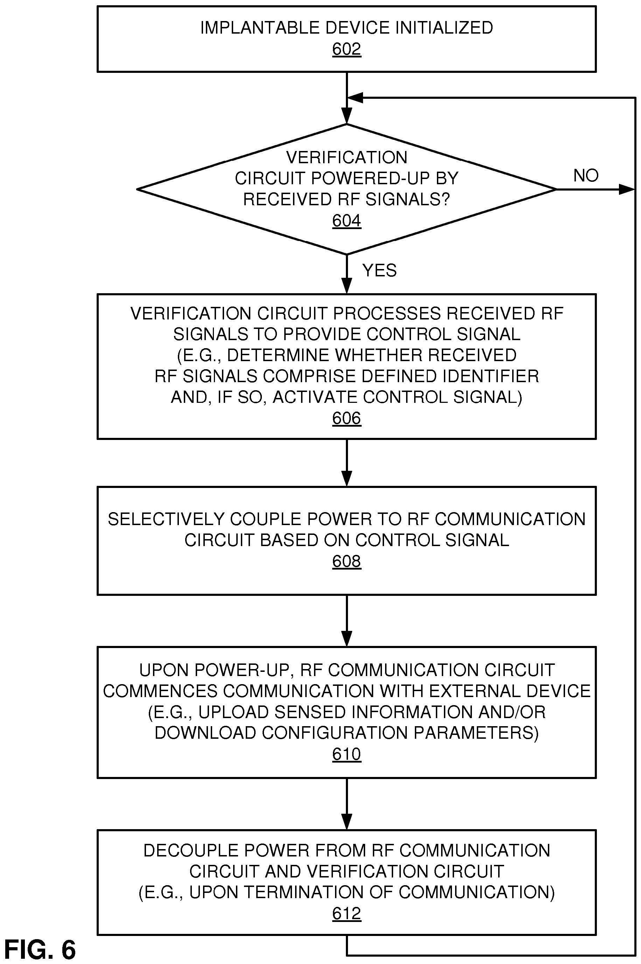

With the above in mind, sample operations that may be performed to provide a communication scheme as taught herein will be described with reference to the flowcharts of FIGs. 5 and 6. FIG. 5 illustrates sample operations that may be performed by an implantable device that power a communication circuit upon detection of a received RF signal (e.g., as in FIG. 2). FIG. 6 illustrates sample operations that may be performed by an implantable device where a received RF signal powers a verification circuit (e.g., as in FIG. 3).

For purposes of illustration, the operations of FIGs. 5 and 6 (or any other operations discussed or taught herein) may be described as being performed by specific components (e.g., components of FIG. 1, 2, 3, 4, 7, 8, or 9). It should be appreciated, however, that these operations may be performed by other types of components and may be performed using a different number of components. It also should be appreciated that one or more of the operations described herein may not be employed in a given implementation.

Referring initially to block 502 of FIG. 5, when the implantable device is initialized upon implant, the RF communication circuit of the implantable device is decoupled from the internal power source (e.g., battery). However, other circuitry (e.g., sensing circuits) that is continually powered by the internal power source will commence normal operations at this time.

As represented by block 504, a detection circuit of the implantable device passively waits for the arrival RF signals. Once RF signals of sufficient magnitude arrive at the implantable device, the RF signals are detected and a signal indicative of this detection is generated. As discussed herein, the detection of the RF signals and the generation of the detection indication signal do not use power from the internal power source.

As represented by block 506, as a result of the detection of the RF signals, power from the internal power source is coupled to the RF communication circuit. For example, the signal generated at block 504 may control a switch that selectively couples the power source to the RF communication circuit.

As represented by block 508, upon power-up, the RF communication circuit commences processing received RF signals. For example, the RF communication circuit may "sniff" for additional RF signals and, if applicable, verify a communication link to an external device (e.g., upon verification of a received key).

As represented by block 510, in the event it is determined that an external device (e.g., an authorized programmer) is attempting to communicate with the implantable device, the implantable device commence communication with the external device to, for example, upload information (e.g., sensed information, parameters, etc.) and/or download information (e.g., commands, parameters, etc.).

As represented by block 512, at some point in time, power is decoupled from the RF communication circuit. For example, upon termination of the communication between the external device and the implantable device, the implantable device may return to the state where it passively waits for the arrival of RF signals (as represented by the arrow from block 512 to block 504).

Referring now to FIG. 6, as represented by block 602, the RF communication circuit of the implantable device is decoupled from the internal power source (e.g., battery) at initialization. Again, however, other circuitry (e.g., sensing circuits) that is continually powered by the internal power source will commence normal operations.

As represented by block 604, a verification circuit of the implantable device passively waits for the arrival of RF signals. Once RF signals of sufficient magnitude arrive at the implantable device, power is obtained from these RF signals to power-up the verification circuit.

As represented by block 606, upon power-up, the verification circuit commences processing received RF signals to determine whether an external device (e.g., an authorized programmer) is attempting to communicate with the implantable device. For example, the verification circuit may "sniff" for additional RF signals and, if applicable, verify a communication link to an external device (e.g., upon verification of a received key). Based on the results of this processing (e.g., the verification), the verification circuit provides a control signal that control whether the RF communication circuit is to be coupled to the internal power source.

As represented by block 608, depending on the current state (e.g., value) of the control signal, power from the internal power source is selectively coupled to the RF communication circuit.

As represented by block 610, in the event the RF communication circuit is powered-up, the RF communication circuit commences communication with the external device. As discussed herein, this communication may involve, for example, uploading information (e.g., sensed information, parameters, etc.) and/or downloading information (e.g., commands, parameters, etc.).

As represented by block 612, at some point in time, power is decoupled from the RF communication circuit and the verification circuit. For example, upon termination of the communication between the external device and the implantable device, the implantable device may return to the state where it passively monitors for RF signals (as represented by the arrow from block 612 to block 604).

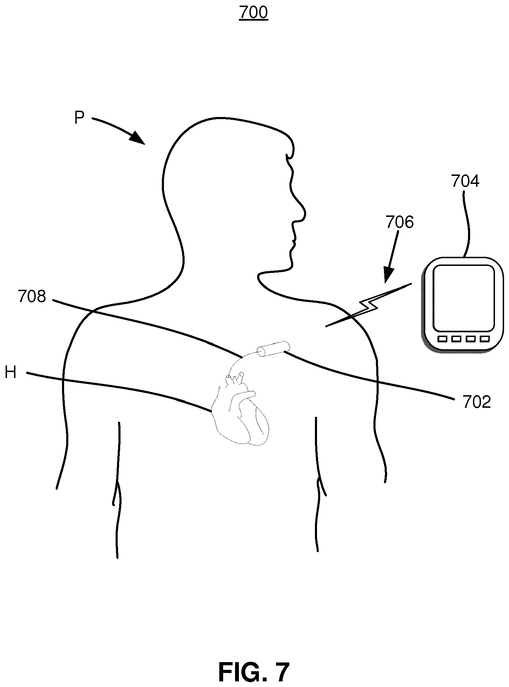

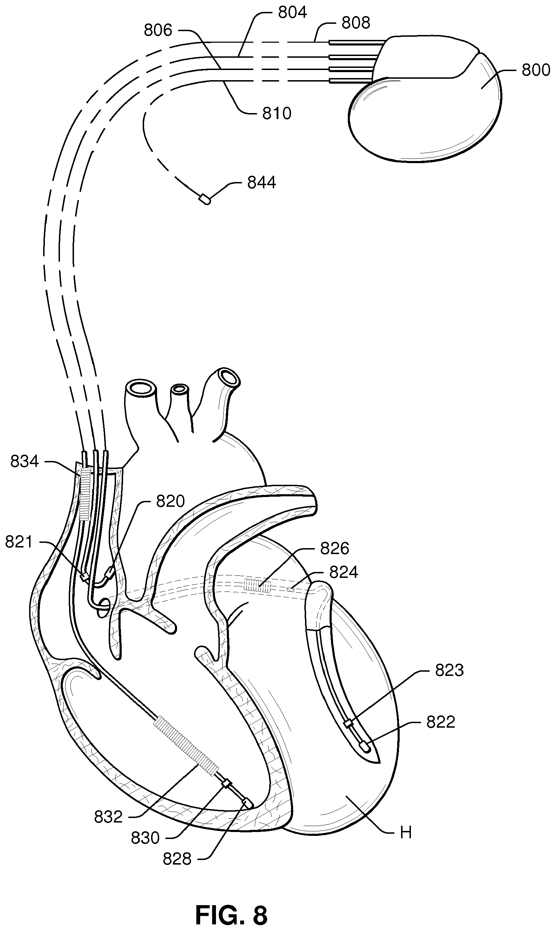

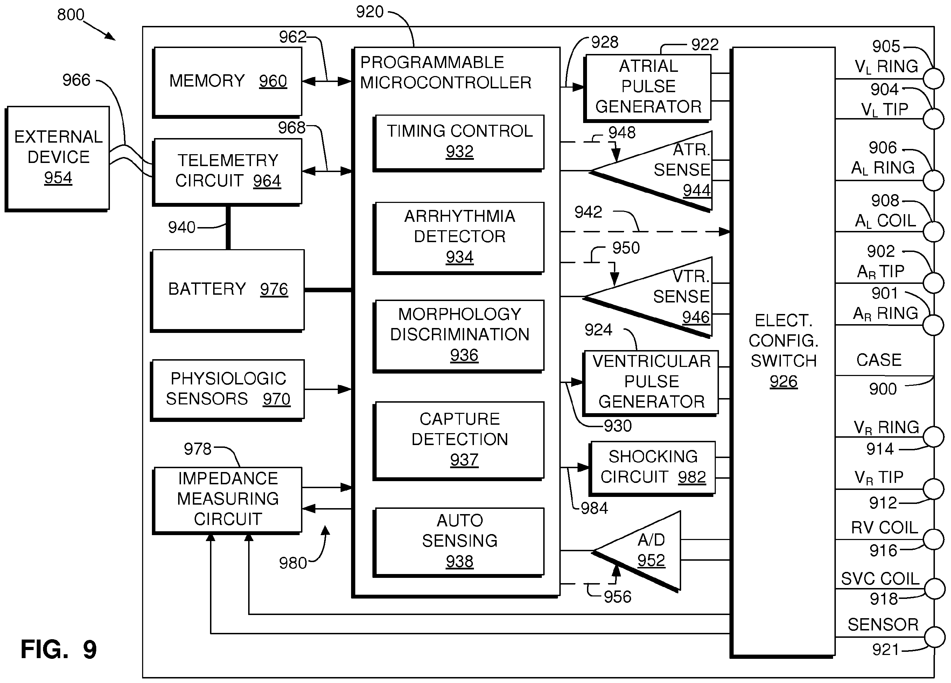

As mentioned above, in some embodiments the teachings herein may be incorporated into an implantable cardiac device. An example of such an implantable cardiac device will be described in more detail in conjunction with FIGs. 7 - 9. FIG. 7 illustrates a sample communication system from a high-level perspective. FIGs. 8 and 9 illustrate sample components of an implantable cardiac device.

FIG. 7 is a simplified diagram of a device 702 (implanted within a patient P) that communicates with a device 704 that is located external to the patient P. The implanted device 702 and the external device 704 communicate with one another via a wireless communication link 706 (as represented by the depicted wireless symbol).

In the illustrated example, the implanted device 702 is an implantable cardiac device including one or more leads 708 that are routed to the heart H of the patient P. For example, the implanted device 702 may be a pacemaker, an implantable cardioverter defibrillator, or some other similar device. It should be appreciated, however, that the implanted device 702 may take other forms.

The external device 704 also may take various forms. For example, the external device 704 may be a base station, a programmer, a home safety monitor, a personal monitor, a follow-up monitor, a wearable monitor, or some other type of device that is configured to communicate with the implanted device 702.

The communication link 706 may be used to transfer information between the devices 702 and 704 in conjunction with various applications such as remote home-monitoring, clinical visits, data acquisition, remote follow-up, and portable or wearable patient monitoring/control systems. For example, when information needs to be transferred between the devices 702 and 704, the patient P moves into a position that is relatively close to the external device 704, or vice versa.

The external device 704 may send information it receives from an implanted device to another device (e.g., that may provide a more convenient means for a physician to review the information). For example, the external device 704 may send the information to a web server (not shown). In this way, the physician may remotely access the information (e.g., by accessing a website). The physician may then review the information uploaded from the implantable device to determine whether medical intervention is warranted.

### Exemplary Cardiac Device

FIGs. 8 and 9 describe exemplary components of an implantable cardiac device that may be used in connection with the various embodiments that are described herein. For example, in some implementations, the disclosed implantable cardiac device may incorporate RF-powered circuitry as discussed herein. As another example, in some implementations, an implantable cardiac device that incorporates RF-powered circuitry as discussed herein may include one or more of the components described in FIGs. 8 and 9. It is to be appreciated and understood that other devices, including those that are not necessarily implantable, may be used in various implementations and that the description below is given, in its specific context, to assist the reader in understanding, with more clarity, the embodiments described herein.

FIG. 8 shows an exemplary implantable cardiac device 800 in electrical communication with a patient's heart H by way of three leads 804, 806, and 808, suitable for delivering multi-chamber stimulation and shock therapy. Bodies of the leads 804, 806, and 808 may be formed of silicone, polyurethane, plastic, or similar biocompatible materials to facilitate implant within a patient. Each lead includes one or more conductors, each of which may couple one or more electrodes incorporated into the lead to a connector on the proximal end of the lead. Each connector, in turn, is configured to couple with a complimentary connector (e.g., implemented within a header) of the device 800.

To sense atrial cardiac signals and to provide right atrial chamber stimulation therapy, the device 800 is coupled to an implantable right atrial lead 804 having, for example, an atrial tip electrode 820, which typically is implanted in the patient's right atrial appendage or septum. FIG. 8 also shows the right atrial lead 804 as having an optional atrial ring electrode 821.

To sense left atrial and ventricular cardiac signals and to provide left chamber pacing therapy, the device 800 is coupled to a coronary sinus lead 806 designed for placement in the coronary sinus region via the coronary sinus for positioning one or more electrodes adjacent to the left ventricle, one or more electrodes adjacent to the left atrium, or both. As used herein, the phrase "coronary sinus region" refers to the vasculature of the left ventricle, including any portion of the coronary sinus, the great cardiac vein, the left marginal vein, the left posterior ventricular vein, the middle cardiac vein, the small cardiac vein or any other cardiac vein accessible by the coronary sinus.

Accordingly, an exemplary coronary sinus lead 806 is designed to receive atrial and ventricular cardiac signals and to deliver left ventricular pacing therapy using, for example, a left ventricular tip electrode 822 and, optionally, a left ventricular ring electrode 823; provide left atrial pacing therapy using, for example, a left atrial ring electrode 824; and provide shocking therapy using, for example, a left atrial coil electrode 826 (or other electrode capable of delivering a shock). For a more detailed description of a coronary sinus lead, the reader is directed to U.S. Patent No. 5,466,254, "Coronary Sinus Lead with Atrial Sensing Capability" (Helland), which is incorporated herein by reference.

The device 800 is also shown in electrical communication with the patient's heart H by way of an implantable right ventricular lead 808 having, in this implementation, a right ventricular tip electrode 828, a right ventricular ring electrode 830, a right ventricular (RV) coil electrode 832 (or other electrode capable of delivering a shock), and a superior vena cava (SVC) coil electrode 834 (or other electrode capable of delivering a shock). Typically, the right ventricular lead 808 is transvenously inserted into the heart H to place the right ventricular tip electrode 828 in the right ventricular apex so that the RV coil electrode 832 will be positioned in the right ventricle and the SVC coil electrode 834 will be positioned in the superior vena cava. Accordingly, the right ventricular lead 808 is capable of sensing or receiving cardiac signals, and delivering stimulation in the form of pacing and shock therapy to the right ventricle.

The device 800 is also shown in electrical communication with a lead 810 including one or more components 844 such as a physiologic sensor. The component 844 may be positioned in, near or remote from the heart.

It should be appreciated that the device 800 may connect to leads other than those specifically shown. In addition, the leads connected to the device 800 may include components other than those specifically shown. For example, a lead may include other types of electrodes, sensors or devices that serve to otherwise interact with a patient or the surroundings.

FIG. 9 depicts an exemplary, simplified block diagram illustrating sample components of the device 800. The device 800 may be adapted to treat both fast and slow arrhythmias with stimulation therapy, including cardioversion, defibrillation, and pacing stimulation. While a particular multi-chamber device is shown, it is to be appreciated and understood that this is done for illustration purposes. Thus, the techniques and methods described below can be implemented in connection with any suitably configured or configurable device. Accordingly, one of skill in the art could readily duplicate, eliminate, or disable the appropriate circuitry in any desired combination to provide a device capable of treating the appropriate chamber(s) with, for example, cardioversion, defibrillation, and pacing stimulation.

A housing 900 for the device 800 is often referred to as the "can", "case" or "case electrode", and may be programmably selected to act as the return electrode for all "unipolar" modes. The housing 900 may further be used as a return electrode alone or in combination with one or more of the coil electrodes 826, 832 and 834 for shocking purposes. The housing 900 may be constructed of a biocompatible material (e.g., titanium) to facilitate implant within a patient.

The housing 900 further includes a connector (not shown) having a plurality of terminals 901, 902, 904, 905, 906, 908, 912, 914, 916 and 918 (shown schematically and, for convenience, the names of the electrodes to which they are connected are shown next to the terminals). The connector may be configured to include various other terminals (e.g., terminal 921 coupled to a sensor or some other component) depending on the requirements of a given application.

To achieve right atrial sensing and pacing, the connector includes, for example, a right atrial tip terminal (AR TIP) 902 adapted for connection to the right atrial tip electrode 820. A right atrial ring terminal (AR RING) 901 may also be included and adapted for connection to the right atrial ring electrode 821. To achieve left chamber sensing, pacing, and shocking, the connector includes, for example, a left ventricular tip terminal (VL TIP) 904, a left ventricular ring terminal (VL RING) 905, a left atrial ring terminal (AL RING) 906, and a left atrial shocking terminal (AL COIL) 908, which are adapted for connection to the left ventricular tip electrode 822, the left ventricular ring electrode 823, the left atrial ring electrode 824, and the left atrial coil electrode 826, respectively.

To support right chamber sensing, pacing, and shocking, the connector further includes a right ventricular tip terminal (VR TIP) 912, a right ventricular ring terminal (VR RING) 914, a right ventricular shocking terminal (RV COIL) 916, and a superior vena cava shocking terminal (SVC COIL) 918, which are adapted for connection to the right ventricular tip electrode 828, the right ventricular ring electrode 830, the RV coil electrode 832, and the SVC coil electrode 834, respectively.

At the core of the device 800 is a programmable microcontroller 920 that controls the various modes of stimulation therapy. As is well known in the art, microcontroller 920 typically includes a microprocessor, or equivalent control circuitry, designed specifically for controlling the delivery of stimulation therapy, and may further include memory such as RAM, ROM and flash memory, logic and timing circuitry, state machine circuitry, and I/O circuitry. Typically, microcontroller 920 includes the ability to process or monitor input signals (data or information) as controlled by a program code stored in a designated block of memory. The type of microcontroller is not critical to the described implementations. Rather, any suitable microcontroller 920 may be used that carries out the functions described herein. The use of microprocessor-based control circuits for performing timing and data analysis functions are well known in the art.

Representative types of control circuitry that may be used in connection with the described embodiments can include the microprocessor-based control system of U.S. Patent No. 4,940,052 (Mann et al.), the state-machine of U.S. Patent Nos. 4,712,555 (Thornander et al.) and 4,944,298 (Sholder), all of which are incorporated by reference herein. For a more detailed description of the various timing intervals that may be used within the device and their inter-relationship, see U.S. Patent 4,788,980 (Mann et al.), also incorporated herein by reference.

FIG. 9 also shows an atrial pulse generator 922 and a ventricular pulse generator 924 that generate pacing stimulation pulses for delivery by the right atrial lead 804, the coronary sinus lead 806, the right ventricular lead 808, or some combination of these leads via an electrode configuration switch 926. It is understood that in order to provide stimulation therapy in each of the four chambers of the heart, the atrial and ventricular pulse generators 922 and 924 may include dedicated, independent pulse generators, multiplexed pulse generators, or shared pulse generators. The pulse generators 922 and 924 are controlled by the microcontroller 920 via appropriate control signals 928 and 930, respectively, to trigger or inhibit the stimulation pulses.

Microcontroller 920 further includes timing control circuitry 932 to control the timing of the stimulation pulses (e.g., pacing rate, atrio-ventricular (A-V) delay, atrial interconduction (A-A) delay, or ventricular interconduction (V-V) delay, etc.) or other operations, as well as to keep track of the timing of refractory periods, blanking intervals, noise detection windows, evoked response windows, alert intervals, marker channel timing, etc., as known in the art.

Microcontroller 920 further includes an arrhythmia detector 934. The arrhythmia detector 934 may be utilized by the device 800 for determining desirable times to administer various therapies. The arrhythmia detector 934 may be implemented, for example, in hardware as part of the microcontroller 920, or as software/firmware instructions programmed into the device 800 and executed on the microcontroller 920 during certain modes of operation.

Microcontroller 920 may include a morphology discrimination module 936, a capture detection module 937 and an auto sensing module 938. These modules are optionally used to implement various exemplary recognition algorithms or methods. The aforementioned components may be implemented, for example, in hardware as part of the microcontroller 920, or as software/firmware instructions programmed into the device 800 and executed on the microcontroller 920 during certain modes of operation.

The electrode configuration switch 926 includes a plurality of switches for connecting the desired terminals (e.g., that are connected to electrodes, coils, sensors, etc.) to the appropriate I/O circuits, thereby providing complete terminal and, hence, electrode programmability. Accordingly, switch 926, in response to a control signal 942 from the microcontroller 920, may be used to determine the polarity of the stimulation pulses (e.g., unipolar, bipolar, combipolar, etc.) by selectively closing the appropriate combination of switches (not shown) as is known in the art.

Atrial sensing circuits (ATR. SENSE) 944 and ventricular sensing circuits (VTR. SENSE) 946 may also be selectively coupled to the right atrial lead 804, coronary sinus lead 806, and the right ventricular lead 808, through the switch 926 for detecting the presence of cardiac activity in each of the four chambers of the heart. Accordingly, the atrial and ventricular sensing circuits 944 and 946 may include dedicated sense amplifiers, multiplexed amplifiers, or shared amplifiers. Switch 926 determines the "sensing polarity" of the cardiac signal by selectively closing the appropriate switches, as is also known in the art. In this way, the clinician may program the sensing polarity independent of the stimulation polarity. The sensing circuits (e.g., circuits 944 and 946) are optionally capable of obtaining information indicative of tissue capture.

Each sensing circuit 944 and 946 preferably employs one or more low power, precision amplifiers with programmable gain, automatic gain control, bandpass filtering, a threshold detection circuit, or some combination of these components, to selectively sense the cardiac signal of interest. The automatic gain control enables the device 800 to deal effectively with the difficult problem of sensing the low amplitude signal characteristics of atrial or ventricular fibrillation.

The outputs of the atrial and ventricular sensing circuits 944 and 946 are connected to the microcontroller 920, which, in turn, is able to trigger or inhibit the atrial and ventricular pulse generators 922 and 924, respectively, in a demand fashion in response to the absence or presence of cardiac activity in the appropriate chambers of the heart. Furthermore, as described herein, the microcontroller 920 is also capable of analyzing information output from the sensing circuits 944 and 946, a data acquisition system 952, or both. This information may be used to determine or detect whether and to what degree tissue capture has occurred and to program a pulse, or pulses, in response to such determinations. The sensing circuits 944 and 946, in turn, receive control signals over signal lines 948 and 950, respectively, from the microcontroller 920 for purposes of controlling the gain, threshold, polarization charge removal circuitry (not shown), and the timing of any blocking circuitry (not shown) coupled to the inputs of the sensing circuits 944 and 946 as is known in the art.

For arrhythmia detection, the device 800 utilizes the atrial and ventricular sensing circuits 944 and 946 to sense cardiac signals to determine whether a rhythm is physiologic or pathologic. It should be appreciated that other components may be used to detect arrhythmia depending on the system objectives. In reference to arrhythmias, as used herein, "sensing" is reserved for the noting of an electrical signal or obtaining data (information), and "detection" is the processing (analysis) of these sensed signals and noting the presence of an arrhythmia.

Timing intervals between sensed events (e.g., P-waves, R-waves, and depolarization signals associated with fibrillation) may be classified by the arrhythmia detector 934 of the microcontroller 920 by comparing them to a predefined rate zone limit (e.g., bradycardia, normal, low rate VT, high rate VT, and fibrillation rate zones) and various other characteristics (e.g., sudden onset, stability, physiologic sensors, and morphology, etc.) in order to determine the type of remedial therapy that is needed (e.g., bradycardia pacing, anti-tachycardia pacing, cardioversion shocks or defibrillation shocks, collectively referred to as "tiered therapy"). Similar rules may be applied to the atrial channel to determine if there is an atrial tachyarrhythmia or atrial fibrillation with appropriate classification and intervention.

Cardiac signals or other signals may be applied to inputs of an analog-to-digital (A/D) data acquisition system 952. The data acquisition system 952 is configured (e.g., via signal line 956) to acquire intracardiac electrogram ("IEGM") signals or other signals, convert the raw analog data into a digital signal, and store the digital signals for later processing, for telemetric transmission to an external device 954, or both. For example, the data acquisition system 952 may be coupled to the right atrial lead 804, the coronary sinus lead 806, the right ventricular lead 808 and other leads through the switch 926 to sample cardiac signals across any pair of desired electrodes.

The data acquisition system 952 also may be coupled to receive signals from other input devices. For example, the data acquisition system 952 may sample signals from a physiologic sensor 970 or other components shown in FIG. 9 (connections not shown).

The microcontroller 920 is further coupled to a memory 960 by a suitable data/address bus 962, wherein the programmable operating parameters used by the microcontroller 920 are stored and modified, as required, in order to customize the operation of the device 800 to suit the needs of a particular patient. Such operating parameters define, for example, pacing pulse amplitude, pulse duration, electrode polarity, rate, sensitivity, automatic features, arrhythmia detection criteria, and the amplitude, waveshape and vector of each shocking pulse to be delivered to the patient's heart H within each respective tier of therapy. One feature of the described embodiments is the ability to sense and store a relatively large amount of data (e.g., from the data acquisition system 952), which data may then be used for subsequent analysis to guide the programming of the device 800.

Advantageously, the operating parameters of the implantable device 800 may be non-invasively programmed into the memory 960 through a telemetry circuit 964 in telemetric communication via communication link 966 with the external device 954, such as a programmer, transtelephonic transceiver, a diagnostic system analyzer or some other device. The microcontroller 920 activates the telemetry circuit 964 with a control signal (e.g., via bus 968). The telemetry circuit 964 advantageously allows intracardiac electrograms and status information relating to the operation of the device 800 (as contained in the microcontroller 920 or memory 960) to be sent to the external device 954 through an established communication link 966.

The device 800 can further include one or more physiologic sensors 970. In some embodiments the device 800 may include a "rate-responsive" sensor that may provide, for example, information to aid in adjustment of pacing stimulation rate according to the exercise state of the patient. One or more physiologic sensors 970 (e.g., a pressure sensor) may further be used to detect changes in cardiac output, changes in the physiological condition of the heart, or diurnal changes in activity (e.g., detecting sleep and wake states). Accordingly, the microcontroller 920 responds by adjusting the various pacing parameters (such as rate, A-V Delay, V-V Delay, etc.) at which the atrial and ventricular pulse generators 922 and 924 generate stimulation pulses.

While shown as being included within the device 800, it is to be understood that a physiologic sensor 970 may also be external to the device 800, yet still be implanted within or carried by the patient. Examples of physiologic sensors that may be implemented in conjunction with the device 800 include sensors that sense respiration rate, pH of blood, ventricular gradient, oxygen saturation, blood pressure and so forth. Another sensor that may be used is one that detects activity variance, wherein an activity sensor is monitored diurnally to detect the low variance in the measurement corresponding to the sleep state. For a more detailed description of an activity variance sensor, the reader is directed to U.S. Patent No. 5,476,483 (Bornzin et al.), which patent is hereby incorporated by reference.

The one or more physiologic sensors 970 may optionally include one or more of components to help detect movement (via, e.g., a position sensor or an accelerometer) and minute ventilation (via an MV sensor) in the patient. Signals generated by the position sensor and MV sensor may be passed to the microcontroller 920 for analysis in determining whether to adjust the pacing rate, etc. The microcontroller 920 may thus monitor the signals for indications of the patient's position and activity status, such as whether the patient is climbing up stairs or descending down stairs or whether the patient is sitting up after lying down.

The device 800 additionally includes a battery 976 that provides operating power to all of the circuits shown in FIG. 9. For a device 800 which employs shocking therapy, the battery 976 is capable of operating at low current drains (e.g., preferably less than 10 µA) for long periods of time, and is capable of providing high-current pulses (for capacitor charging) when the patient requires a shock pulse (e.g., preferably, in excess of 2 A, at voltages above 200 V, for periods of 10 seconds or more). The battery 976 also desirably has a predictable discharge characteristic so that elective replacement time can be detected. Accordingly, the device 800 preferably employs lithium or other suitable battery technology.

The device 800 can further include magnet detection circuitry (not shown), coupled to the microcontroller 920, to detect when a magnet is placed over the device 800. A magnet may be used by a clinician to perform various test functions of the device 800 and to signal the microcontroller 920 that the external device 954 is in place to receive data from or transmit data to the microcontroller 920 through the telemetry circuit 964.

The device 800 further includes an impedance measuring circuit 978 that is enabled by the microcontroller 920 via a control signal 980. The known uses for an impedance measuring circuit 978 include, but are not limited to, lead impedance surveillance during the acute and chronic phases for proper performance, lead positioning or dislodgement; detecting operable electrodes and automatically switching to an operable pair if dislodgement occurs; measuring respiration or minute ventilation; measuring thoracic impedance for determining shock thresholds; detecting when the device 800 has been implanted; measuring stroke volume; and detecting the opening of heart valves, etc. The impedance measuring circuit 978 is advantageously coupled to the switch 926 so that any desired electrode may be used.

In the case where the device 800 is intended to operate as an implantable cardioverter/defibrillator (ICD) device, it detects the occurrence of an arrhythmia, and automatically applies an appropriate therapy to the heart aimed at terminating the detected arrhythmia. To this end, the microcontroller 920 further controls a shocking circuit 982 by way of a control signal 984. The shocking circuit 982 generates shocking pulses of low (e.g., up to 0.5 J), moderate (e.g., 0.5 J to 10 J), or high energy (e.g., 11 J to 40 J), as controlled by the microcontroller 920. Such shocking pulses are applied to the patient's heart H through, for example, two shocking electrodes and as shown in this embodiment, selected from the left atrial coil electrode 826, the RV coil electrode 832 and the SVC coil electrode 834. As noted above, the housing 900 may act as an active electrode in combination with the RV coil electrode 832, as part of a split electrical vector using the SVC coil electrode 834 or the left atrial coil electrode 826 (i.e., using the RV electrode as a common electrode), or in some other arrangement.

Cardioversion level shocks are generally considered to be of low to moderate energy level (so as to minimize pain felt by the patient), be synchronized with an R-wave, pertain to the treatment of tachycardia, or some combination of the above. Defibrillation shocks are generally of moderate to high energy level (i.e., corresponding to thresholds in the range of 5 J to 40 J), delivered asynchronously (since R-waves may be too disorganized), and pertaining to the treatment of fibrillation. Accordingly, the microcontroller 920 is capable of controlling the synchronous or asynchronous delivery of the shocking pulses.

As mentioned above, the device 800 may include one or more components that provide RF-powered functionality as taught herein. For example, the telemetry circuit 964 may include a communication circuit along with a detection circuit or a verification circuit as taught herein. Here, the power to be coupled to the communication circuit may be obtained from the battery 976 via a power path 940. Also, one or more of the sense circuit 944, the sense circuit 946, the sensors 970, or the data acquisition system 952 may acquire information that is to be sent through the use of an RF-powered communication circuit. The data described above may be stored in the data memory 960. In addition, the microcontroller 920 (e.g., a processor providing signal processing functionality) also may implement or support at least a portion of the processing functionality discussed herein.

It should be appreciated that various modifications may be incorporated into the disclosed embodiments based on the teachings herein. For example, the structure and functionality taught herein may be incorporated into types of devices other than the specific types of devices described above. In addition, different types of detection circuits or verification circuits may be employed in different embodiments.

Also, the circuitry discussed herein may be implemented in different ways in different embodiments. For example, in some embodiments, a rectification circuit is implemented using at least one conventional diode rectifier. In other embodiments, a rectification circuit is implemented using a synchronous rectifier. In a typical case, a synchronous rectifier employs field effect transistors (FETs) to rectify the RF alternating current (AC) signal by steering the alternating current into direct current (DC). The resulting signal may then be filtered using conventional filtering.

It should be appreciated from the above that the various structures and functions described herein may be incorporated into a variety of apparatuses (e.g., a stimulation device, a lead, a monitoring device, a satellite device, etc.) and implemented in a variety of ways. Different embodiments of such an apparatus may include a variety of hardware and software processing components. In some embodiments, hardware components such as processors, controllers, state machines, logic, or some combination of these components, may be used to implement the described components or circuits.

In some embodiments, code including instructions (e.g., software, firmware, middleware, etc.) may be executed on one or more processing devices to implement one or more of the described functions or components. The code and associated components (e.g., data structures and other components used by the code or used to execute the code) may be stored in an appropriate data memory that is readable by a processing device (e.g., commonly referred to as a computer-readable medium).

Moreover, some of the operations described herein may be performed by a device that is located externally with respect to the body of the patient. For example, an implanted device may send raw data or processed data to an external device that then performs the necessary processing.

The components and functions described herein may be connected or coupled in many different ways. The manner in which this is done may depend, in part, on whether and how the components are separated from the other components. In some embodiments some of the connections or couplings represented by the lead lines in the drawings may be in an integrated circuit, on a circuit board or implemented as discrete wires or in other ways.

The signals discussed herein may take various forms. For example, in some embodiments a signal may comprise electrical signals transmitted over a wire, light pulses transmitted through an optical medium such as an optical fiber or air, or RF waves transmitted through a medium such as air, and so on. In addition, a plurality of signals may be collectively referred to as a signal herein. The signals discussed above also may take the form of data. For example, in some embodiments an application program may send a signal to another application program. Such a signal may be stored in a data memory.

Moreover, the recited order of the blocks in the processes disclosed herein is simply an example of a suitable approach. Thus, operations associated with such blocks may be rearranged while remaining within the scope of the present disclosure. Similarly, the accompanying method claims present operations in a sample order, and are not necessarily limited to the specific order presented.

Also, it should be understood that any reference to elements herein using a designation such as "first," "second," and so forth does not generally limit the quantity or order of those elements. Rather, these designations may be used herein as a convenient method of distinguishing between two or more different elements or instances of an element. Thus, a reference to first and second elements does not mean that only two elements may be employed there or that the first element must precede the second element in some manner. Also, unless stated otherwise a set of elements may comprise one or more elements. In addition, terminology of the form "at least one of A, B, or C" or "one or more of A, B, or C" or "at least one of the group consisting of A, B, and C" used in the description or the claims means "A or B or C or any combination of these elements."

As used herein, the term "determining" encompasses a wide variety of actions. For example, "determining" may include calculating, computing, processing, deriving, investigating, looking up (e.g., looking up in a table, a database or another data structure), ascertaining, and the like. Also, "determining" may include receiving (e.g., receiving information), accessing (e.g., accessing data in a memory), and the like. Also, "determining" may include resolving, selecting, choosing, establishing, and the like.

While certain embodiments have been described above in detail and shown in the accompanying drawings, it is to be understood that such embodiments are merely illustrative of and not restrictive of the teachings herein. In particular, it should be recognized that the teachings herein apply to a wide variety of apparatuses and methods. It will thus be recognized that various modifications may be made to the illustrated embodiments or other embodiments, without departing from the scope of the invention as defined by the claims.

## Claims

1. An implantable device, comprising:
a power source;
a radiofrequency communication circuit;
a circuit configured to selectively couple power from the power source to the radiofrequency communication circuit based on a control signal;
an antenna; and
a verification circuit coupled to the antenna, and configured to:
obtain power from a first portion of radiofrequency signals received via the antenna,
determine, based on a second portion of the received radiofrequency signals, whether the received radiofrequency signals comprise a defined identifier, and
based on the determination, provide the control signal.

2. The device of claim 1, wherein the verification circuit is further configured to:
adjust a value of the control signal to selectively enable the coupling of power from the power source to the radiofrequency communication circuit based on the determination.

3. The device of claim 1, wherein:
the verification circuit is activated upon receiving the radiofrequency signals via the antenna;
upon activation, the verification circuit determines whether radiofrequency communication is to be established with an external device; and
the verification circuit controls the control signal based on the determination.

4. The device of claim 1, wherein the verification circuit is configured to:
store at least a portion of the obtained power in an energy storage device; and
use the stored power to provide the control signal.

5. The device of claim 1, wherein at least one of:
a) the circuit configured to selectively couple power is further configured to decouple the power source from the radiofrequency communication circuit based on receipt of a control signal; and
b) the verification circuit comprises a rectification circuit configured to rectify the received radio frequency signals to provide the power for the verification circuit.

6. The device of claim 1, wherein the device comprises at least one other circuit, and the power source comprises a battery that provides power to the at least one other circuit via a coupling that does not include the verification circuit, optionally wherein the at least one other circuit comprises:
a sensing circuit configured to sense a condition of a patient to provide sensed information;
a processing circuit configured to process the sensed information and provide the processed information to the radiofrequency communication circuit for transmission to an external device.

7. The device of claim 1, wherein:
the device has a diameter on the order of 7 millimeters or less; and
the antenna comprises a coil that has a diameter on the order of 4 millimeters or less.

8. A method of communication using the implantable device of any of claims 1 to 7, comprising:
obtaining power from a first portion of radiofrequency signals received via an antenna;
using the obtained power to power a verification circuit;
processing a second portion of the received radiofrequency signals at the verification circuit to determine whether the received radiofrequency signals comprise a defined identifier and, based on the determination, provide a control signal; and
selectively coupling power from a power source to a radiofrequency communication circuit based on the control signal.

## Patentansprüche

1. Implantierbare Vorrichtung, umfassend:
eine Stromquelle;
einen Hochfrequenzkommunikationsschaltkreis;
einen Schaltkreis, der konfiguriert ist, um einen Strom von der Stromquelle aufgrund eines Steuersignals selektiv mit dem Hochfrequenzkommunikationsschaltkreis zu verbinden;
eine Antenne; und
einen Prüfschaltkreis, der mit der Antenne verbunden ist und der konfiguriert ist zum:
Erhalten eines Stroms aus einem ersten Anteil von Hochfrequenzsignalen, die über die Antenne empfangen wurden,
Ermitteln aufgrund eines zweiten Anteils der empfangenen Hochfrequenzsignale, ob die empfangenen Hochfrequenzsignale eine definierte Kennung umfassen, und
aufgrund des Ermittelns, Bereitstellen des Steuersignals.

2. Vorrichtung nach Anspruch 1, wobei der Prüfschaltkreis außerdem konfiguriert ist zum:
Einstellen eines Werts des Steuersignals, um das Verbinden des Stroms von der Stromquelle mit dem Hochfrequenzkommunikationsschaltkreis aufgrund des Ermittelns selektiv zu ermöglichen.

3. Vorrichtung nach Anspruch 1, wobei:
der Prüfschaltkreis nach dem Empfangen der Hochfrequenzsignale über die Antenne aktiviert wird;
der Prüfschaltkreis nach dem Aktivieren ermittelt, ob die Hochfrequenzkommunikation mit einer externen Vorrichtung eingerichtet werden soll; und
der Prüfschaltkreis das Steuersignal aufgrund des Ermittelns steuert.

4. Vorrichtung nach Anspruch 1, wobei der Prüfschaltkreis konfiguriert ist zum:
Speichern mindestens eines Teils des erhaltenen Stroms in einer Energiespeichervorrichtung; und
Verwenden des gespeicherten Stroms, um das Steuersignal bereitzustellen.

5. Vorrichtung nach Anspruch 1, wobei mindestens entweder
a) der Schaltkreis, der konfiguriert ist, um einen Strom selektiv zu verbinden, außerdem konfiguriert ist, um den Strom aufgrund eines Empfangs eines Steuersignals von dem Hochfrequenzkommunikationsschaltkreis zu trennen; oder
b) der Prüfschaltkreis einen Gleichrichterschaltkreis umfasst, der konfiguriert ist, um die empfangenen Hochfrequenzsignale gleichzurichten, um den Strom für den Gleichrichterschaltkreis bereitzustellen.

6. Vorrichtung nach Anspruch 1, wobei die Vorrichtung mindestens einen weiteren Schaltkreis umfasst und wobei die Stromquelle eine Batterie umfasst, die den mindestens einen weiteren Schaltkreis über eine Verbindung mit Strom versorgt, die den Prüfschaltkreis nicht enthält, wobei der mindestens eine weitere Schaltkreis optional umfasst:
einen Erfassungsschaltkreis, der konfiguriert ist, um eine Bedingung eines Patienten zu erfassen, um erfasste Informationen bereitzustellen;
einen Verarbeitungsschaltkreis, der konfiguriert ist, um die erfassten Informationen zu Verarbeiten und um dem Hochfrequenzkommunikationsschaltkreis die verarbeiteten Informationen für eine Übertragung an eine externe Vorrichtung bereitzustellen.

7. Vorrichtung nach Anspruch 1, wobei:
die Vorrichtung einen Durchmesser in der Größenordnung von 7 mm oder weniger aufweist; und
die Antenne eine Spule umfasst, die einen Durchmesser in der Größenordnung von 4 mm oder weniger aufweist.

8. Kommunikationsverfahren zum Verwenden der implantierbaren Vorrichtung nach einem der Ansprüche 1 bis 7, umfassend:
Erhalten eines Stroms aus einem ersten Anteil von Hochfrequenzsignalen, die über eine Antenne empfangen wurden;
Verwenden des erhaltenen Stroms, um einen Prüfschaltkreis mit Strom zu versorgen;
Verarbeiten eines zweiten Anteils der empfangenen Hochfrequenzsignale in dem Prüfschaltkreis, um zu ermitteln, ob die empfangenen Hochfrequenzsignale eine definierte Kennung umfassen, und aufgrund des Ermittelns, Bereitstellen eines Steuersignals; und
aufgrund des Steuersignals, selektives Verbinden eines Stroms von einer Stromquelle mit dem Hochfrequenzkommunikationsschaltkreis.

## Revendications

1. Dispositif implantable, comprenant :
une source d'énergie ;
un circuit de communication radiofréquence ;
un circuit configuré pour coupler de façon sélective l'énergie provenant de la source d'énergie au circuit de communication radiofréquence en se basant sur un signal de commande ;
une antenne ; et
un circuit de vérification couplé à l'antenne et configuré :
pour obtenir de l'énergie d'une première partie de signaux radiofréquences reçus par le biais de l'antenne ;
pour déterminer, en se basant sur une seconde partie des signaux radiofréquences reçus, si les signaux radiofréquences reçus comprennent un identifiant défini et
en se basant sur la détermination, pour fournir le signal de commande.

2. Dispositif selon la revendication 1, dans lequel le circuit de vérification est en outre configuré :
pour ajuster une valeur du signal de commande pour permettre de façon sélective le couplage de l'énergie provenant de la source d'énergie au circuit de communication radiofréquence en se basant sur la détermination.

3. Dispositif selon la revendication 1, dans lequel :
le circuit de vérification est activé lors de la réception des signaux radiofréquences par le biais de l'antenne ;
lors de l'activation, le circuit de vérification détermine si une communication radiofréquence doit être établie avec un dispositif externe ; et
le circuit de vérification commande le signal de commande en se basant sur la détermination.

4. Dispositif selon la revendication 1, dans lequel le circuit de vérification est configuré :
pour stocker au moins une partie de l'énergie obtenue dans un dispositif de stockage d'énergie et
pour utiliser l'énergie stockée pour fournir le signal de commande.

5. Dispositif selon la revendication 1, dans lequel
a) le circuit configuré pour coupler de façon sélective de l'énergie est en outre configuré pour désolidariser la source d'énergie du circuit de communication radiofréquence en se basant sur la réception d'un signal de commande ; et/ou
b) le circuit de vérification comprend un circuit de redressement configuré pour redresser les signaux radiofréquences reçus pour fournir l'énergie pour le circuit de vérification.

6. Dispositif selon la revendication 1, dans lequel le dispositif comprend au moins un autre circuit et la source d'énergie comprend une batterie qui fournit de l'énergie au ou aux autres circuits par le biais d'un couplage qui ne comprend pas le circuit de vérification, facultativement dans lequel le ou les autres circuits comprennent :
un circuit de détection configuré pour détecter un état d'un patient pour fournir des informations détectées ;
un circuit de traitement configuré pour traiter les informations détectées et fournir les informations traitées au circuit de communication radiofréquence pour une transmission à un dispositif externe.

7. Dispositif selon la revendication 1, dans lequel :
le dispositif présente un diamètre de l'ordre de 7 millimètres ou moins ; et
l'antenne comprend une bobine qui présente un diamètre de l'ordre de 4 millimètres ou moins.

8. Procédé de communication à l'aide du dispositif implantable selon l'une quelconque des revendications 1 à 7, consistant :
à obtenir de l'énergie d'une première partie de signaux radiofréquences reçus par le biais d'une antenne ;
à utiliser l'énergie obtenue pour alimenter un circuit de vérification ;
à traiter une seconde partie des signaux radiofréquences reçus au niveau du circuit de vérification pour déterminer si les signaux radiofréquences reçus comprennent un identifiant défini et, en se basant sur la détermination, à fournir un signal de commande ; et
à coupler de manière sélective de l'énergie provenant d'une source d'énergie à un circuit de communication radiofréquence en se basant sur le signal de commande.
